# EUROPÄISCHE PATENTANMELDUNG

(11) **EP 3 453 371 A1**
(43) Veröffentlichungstag der Anmeldung: **13.03.2019**
(21) Anmeldenummer: 18194100.6
(22) Anmeldetag: 12.09.2018
(51) Int. Cl.: A61G 7/018, A61G 7/05, A61B 5/11, A61G 7/005, A61G 7/008, A61G 7/015

(54) **STEUERGERÄT FÜR EIN BETT, ZUM STEUERN VON AKTOREN, INSBESONDERE INNERHALB UND AUSSERHALB DES BETTES**

(30) Priorität: 12.09.2017 DE 102017121099
(71) Anmelder: Wissner-Bosserhoff GmbH, 58739 Wickede (DE)
(72) Erfinder: Bernal, Carlos, 59494 Soest (DE); Makroum, Kheir Eddine, 44287 Dortmund (DE); Bönemann, Burkhard, 58706 Menden (DE)
(74) Vertreter: Schäperklaus, Jochen

(57) **Zusammenfassung**

Steuergerät für ein Bett, zum Steuern von Aktoren, insbesondere innerhalb und außerhalb des Bettes, zum Beispiel
- Motoren zum Einstellen der Liegefläche oder von Teilen der Liegefläche, insbesondere zum Einstellen der Höhe oder der Neigung,
- ein Motor einer Jalousie, eines Rollos, eines Tür- oder Fensteröffners,
- eine Leuchte, insbesondere eine Leuchte einer Unterbettbeleuchtung, eine Leseleuchte, eine Deckenleuchte oder eine Wandleuchte
- eine optische oder akustische Rufeinrichtung,
- ein Anzeigemittel, insbesondere ein Bildschirm,
- Radios oder Fernsehgeräte,
- Telefone oder anderer Telekommunikationseinrichtungen,
- Computer und Computerperipheriegeräten,
wobei das Steuergerät einen Mikrocontroller aufweist, der wenigstens eine Schnittstelle zur Kommunikation mit Sensoren und Aktoren und/oder wenigstens einen Eingang zur Kommunikation mit Sensoren und/oder wenigstens einen Ausgang zur Kommunikation mit Aktoren aufweist.

## Beschreibung

Die vorliegende Erfindung betrifft ein Bett, insbesondere ein medizinisches Bett im Sinne nach EN 60601-2-52, insbesondere ein Krankenbett oder Pflegebett.

Aus dem Dokument DE 20 2016 102 281 U1 ist ein Pflegebett bekannt, dass einen EnOcean-Sensor und einen EnOcean-Aktor aufweist, zum Beispiel um Motoren zum Einstellen der Liegefläche oder von Teilen der Liegefläche, insbesondere zum Einstellen der Höhe oder der Neigung, anzusteuern. Mit dem EnOcean-Sensor können nach dem im Dokument DE 20 2016 102 281 U1 vorgestellten Konzept auch andere EnOcean-Aktoren innerhalb oder außerhalb des Bettes angesteuert werden.

Neben den über einen EnOcean-Aktor ansteuerbaren Funktionen kann es in einem medizinischen Bett oder in der Umgebung eines medizinischen Bettes Komponenten geben, die von einem Bettinsassen über Aktoren angesteuert werden, die keine EnOcean-Aktoren sind. Außerdem gibt es Komponenten, die sowohl von EnOcean-Aktoren angesteuert werden können als auch von nicht EnOcean-Aktoren angesteuert werden, zum Beispiel mittels eines kabelgebundenen Handschalters, wie er in dem Dokument EP 1 647 213 A1 beschrieben ist. Es kann dann der Fall eintreten, dass die Bedienung einer Komponente mittels eines kabelgebundenen Handschalters Vorrang vor einer Bedienung durch den EnOcean-Sensor bzw. EnOcean-Aktor hat. Es muss dann eine Verbindung zwischen der Steuerung durch den Handschalter und der Steuerung durch einen EnOcean-Sensor vorgesehen sein oder eine andere Möglichkeit vorgesehen sein, den Vorrang des Handschalters sicherzustellen. Neben dem in dem Dokument DE 20 2016 102 281 U1 beschriebenen Konzept von EnOcean-Sensoren und EnOcean-Aktoren und den bekannten kabelgebundenen Sensoren und Aktoren können weitere Konzepte für die Bedienung von Komponenten innerhalb und außerhalb von Betten entwickelt und umgesetzt werden. Je mehr Konzepte für die Bedienung von

Je mehr verschiedene Konzepte für die Bedienung von Komponenten in einem Bett verwendet werden, um so komplexer wird die Struktur der Steuerung des Bettes.

Hier setzt die Erfindung an.

Der Erfindung liegt die Aufgabe zu Grunde, den Stand der Technik so zu verbessern, dass verschiedene Konzepte für die Bedienung von Komponenten innerhalb und außerhalb eines Bettes vom Bett auf einfache Art umgesetzt werden können.

Diese Aufgabe wird durch ein Steuergerät für ein Bett mit den Merkmalen des Anspruchs 1 gelöst. Die Erfindung ist danach ein Steuergerät für ein Bett, das sich von anderen Steuerkonzepten dadurch auszeichnet, dass es einen Mikrocontroller aufweist, der wenigstens eine Schnittstelle zur Kommunikation mit Sensoren und Aktoren und/oder wenigstens einen Eingang zur Kommunikation mit wenigstens einem Sensor und/oder wenigstens einen Ausgang zur Kommunikation mit wenigstens einem Aktor aufweist.

Das Steuergerät ist durch den Mikrocontroller mit der wenigstens einen Schnittstelle für eine vorzugsweise bidirektionale Kommunikation mit Sensoren und/oder Aktoren und/oder dem wenigstens einen Eingang zur Kommunikation mit wenigstens einem Sensor und/oder dem wenigstens einen Ausgang zur Kommunikation mit wenigstens einem Aktor offen für verschiedene Kommunikationskonzepte zwischen Sensoren und Aktoren. Von den Sensoren über den oder die Eingänge und/oder der oder den Schnittstellen gelieferte Informationen werden in dem Mikrocontroller verarbeitet, wozu der Mikrocontroller programmiert ist. Der Mikrocontroller erzeugt aufgrund der Informationen von dem oder den Sensoren eine oder mehrere Steueranweisungen oder Stellsignale, die über die wenigstens eine Schnittstelle und/oder den wenigstens einen Eingang an die Aktoren gegeben werden. In dem Mikrocontroller des Steuergerätes können Informationen, die über verschiedene Kommunikationswege von Sensoren bereitgestellt werden, verknüpft werden. Mit dem Mikroprozessor ist man in der Lage, bestimmte Informationen zu Priorisieren, um zum Beispiel bei widersprechenden Informationen eine Lösung zu finden.

Die eine Schnittstelle oder wenigstens eine der Schnittstellen kann eine Funk-Schnittstelle, insbesondere eine EnOcean-Schnittstelle, eine Bluetooth-Schnittstelle oder eine WLAN-Schnittstelle sein. Ein erfindungsgemäßes Steuergerät kann verschiedene Funkschnittstellen aufweisen, zum Beispiel eine oder mehrere Bluetooth-Schnittstellen, eine WLAN-Schnittstelle und/oder eine oder mehrere EnOcean-Schnittstellen.

Die eine Schnittstelle oder die wenigstens eine Schnittstelle des Mikrocontrollers kann wenigstens eine Bus-Schnittstelle, insbesondere eine CAN-Schnittstelle, eine Ethernet-Schnittstelle, KNX-Schnittstelle, eine Insta-Bus-Schnittstelle, USB-Schnittstelle oder eine LIN-Schnittstelle sein. Ein erfindungsgemäßes Steuergerät kann verschiedene Bus-Schnittstellen aufweisen, zum Beispiel eine CAN-Bus-Schnittstelle, eine Ethernet-Schnittstelle, KNX-Schnittstelle, eine Insta-Bus-Schnittstelle, eine USB-Schnittstelle oder eine LIN-Schnittstelle.

Ein Mikrocontroller eines erfindungsgemäßen Steuergerätes kann auch wenigstens eine Funk-Schnittstelle und wenigstens eine Bus-Schnittstelle aufweisen.

Es ist möglich, dass wenigstens an eine Busschnittstelle ein Funkkommunikationsmodul, zum Beispiel ein EnOcean, Bluetooth oder WLAN-Modul des Steuergerätes angeschlossen ist, an den ein Sensor oder ein Aktor anschließbar ist. Mittels eines Funkkommunikationsmoduls kann der Mikrocontroller auch über Funk mit einem Sensor und/oder einem Aktor verbunden sein, wenn er nur eine Bus-Schnittstelle aufweist.

Ebenso ist es möglich, dass an wenigstens eine der Schnittstellen ein Eingangsmodul mit einem Eingang und/oder Ausgangsmodul mit einem Ausgang anschließbar ist. Sollte der Mikrocontroller eines erfindungsgemäßen Steuergerätes keinen Eingang oder Ausgang aufweisen, an dem ein bestimmter Sensor oder ein bestimmter Aktor angeschlossen werden kann, sondern nur wenigstens eine Schnittstelle, kann durch ein Eingangsmodul oder ein Ausgangsmodul, dass an die wenigstens eine Schnittstelle des Mikrocontrollers angeschlossen ist, der bestimmte Sensor oder der bestimmte Aktor angeschlossen werden.

Der an die wenigstens eine Schnittstelle oder den Eingang anschließbare Sensor kann ein Bedienelement, eine Sensoreinrichtung, ein Messwertgeber, eine Bedieneinrichtung, insbesondere ein Handschalter oder ein Smartphone, oder ähnliches sein. Ein Sensor kann im Grunde jede Komponente sein, die eine Information, zum Beispiel einen Messwert, eine Positionsinformation oder eine Anweisung, liefert. Der Sensor kann insbesondere eine Waage, eine Sensormatte, ein Positionssensor, ein Kraftsensor, ein Druckschalter oder ein Helligkeitssensor sein.

Ein Bedienelement, das als Sensor an ein erfindungsgemäßes Steuergerät angeschlossen sein kann, kann ein Notschalter sein, der über einen Eingang oder eine Busschnittstelle mit dem Steuergerät verbunden ist und mit dem ein aufgrund eines über die Funk-Schnittstelle oder eine der Funk-Schnittstellen empfangenes Sensor- oder Bediensignal vom Mikrocontroller erzeugten Signals für einen Aktor, insbesondere für einen Motor zum Einstellen der Liegefläche oder von Teilen der Liegefläche, deaktivierbar ist. Dem Signal von dem Notschalter wird damit zumindest eine Priorität vor einem Signal eingeräumt, das über Funk an das Steuergerät geliefert wird.

Ein erfindungsgemäßes Steuergerät kann so gestaltet sein,
- dass mit dem Steuergerät, insbesondere mit dem Mikrocontroller, ein Aktorsignal erzeugt werden kann, um eine an einen Ausgang oder eine Bus-Schnittstelle angeschlossene Unterbettbeleuchtung einzuschalten, sobald eine Sensormatte, eine Waage, ein Kraftsensor und/oder ein Druckschalter, die bzw. der an einen Eingang, eine Funk-Schnittstelle und/oder eine Bus-Schnittstelle angeschlossen ist, eine Entlastung detektiert und
- dass mit dem Steuergerät, insbesondere mit dem Mikrocontroller, ein Aktorsignal erzeugt werden kann, um eine an einen Ausgang oder eine Bus-Schnittstelle angeschlossene Unterbettbeleuchtung auszuschalten, sobald die Sensormatte, die Waage, der Kraftsensor oder der Druckschalter eine Belastung detektiert.

Eine Entlastung einer Sensormatte, einer Waage, eines Kraftsensors oder eines Druckschalters liegt auch dann vor, wenn mittels der Sensormatte, der Waage, des Kraftsensors oder des Druckschalters eine Bewegung des Bettbewohners erkennbar ist, die zu einem Verlassen des Bettes führen könnte.

Ein erfindungsgemäßes Steuergerät kann insbesondere so gestaltet sein,
- dass mit dem Steuergerät, insbesondere mit dem Mikrocontroller, ein Aktorsignal erzeugt werden kann, um eine an einen Ausgang oder eine Bus-Schnittstelle angeschlossene Unterbettbeleuchtung einzuschalten, sobald ein Sensor, der an einen Eingang, eine Funk-Schnittstelle oder eine Bus-Schnittstelle angeschlossen ist, ein Absenken einer Seitensicherung des Bettes detektiert und
- dass mit dem Steuergerät, insbesondere mit dem Mikrocontroller, ein Aktorsignal erzeugt werden kann, um eine an einen Ausgang oder eine Bus-Schnittstelle angeschlossene Unterbettbeleuchtung auszuschalten, sobald der Sensor ein Hochziehen der Seitensicherung des Bettes detektiert.

Es ist ferner möglich, dass mittels eines erfindungsgemäßen Steuergerätes die Unterbettbeleuchtung nur dann erfolgt, wenn bei abgesenkter Seitensicherung mittels der Sensormatte, der Waage, des Kraftsensors oder des Druckschalters eine Entlastung der Liegefläche detektiert werden kann.

Sollte keine Sensormatte, keine Waage, kein Kraftsensor und kein Druckschalter vorhanden sein, mit dem ein erfindungsgemäßes Steuergerät eine Entlastung der Liegefläche erkennen kann, kann das Einschalten der Unterbettbeleuchtung dadurch ausgelöst werden, wenn die Seitensicherung abgesenkt wird, und dies über einen Sensor an das erfindungsgemäße Steuergerät des Bettes gemeldet wird. Ferner ist es möglich, dass das erfindungsgemäße Steuergerät mit einem Helligkeitssensor verbunden ist, mit dem festgestellt werden kann, ob ohne Einschalten der Unterbettbeleuchtung die Helligkeit im Bereich des Bettes ausreichend für ein sicheres Bewegen eines Bettbewohners vor dem Bett ist.

Das kann bedeuten, dass bei entlasteter Sensormatte, entlasteter Waage, entlastetem Kraftsensor, entlastetem Druckschalter oder abgesenkter Seitensicherung ein Einschalten der Unterbettbeleuchtung durch das Steuergerät, insbesondere mit dem Mikrocontroller, unterbleibt, wenn ein Helligkeitssensor eine Helligkeit oberhalb eines festgelegten Schwellwerts meldet oder signalisiert.

Es ist auch möglich, dass die Unterbettbeleuchtung mittels des Steuergerätes dimmbar ist. Die Helligkeit im Bereich des Bettes könnte dann bei einem mit dem Steuergerät verbundenen Helligkeitssensor geregelt werden, so dass einerseits eine ausreichende Helligkeit für ein sicheres Bewegen des Bettbewohners gegeben ist und andererseits der Bettbewohner durch die Unterbettbeleuchtung nicht so geblendet wird, dass er weiter vom Bett entfernte Gegenstände nicht mehr oder nicht mehr gut wahrnehmen kann.

Es ist möglich, dass der wenigstens eine Eingang und/oder der wenigstens eine Ausgang des Steuergerätes konfiguriert werden kann. Ebenso ist es möglich, dass das Steuergerät derart konfiguriert werden kann, dass konfigurierbar ist, welche Aktoren und Sensoren über Schnittstellen mit dem Steuergerät verbindbar oder verbunden sind.

Weitere Merkmale und Vorteile der vorliegenden Erfindung werden deutlich anhand der nachfolgenden Beschreibung eines bevorzugten Ausführungsbeispiels unter Bezugnahme auf die beiliegende Abbildung. Darin zeigt:

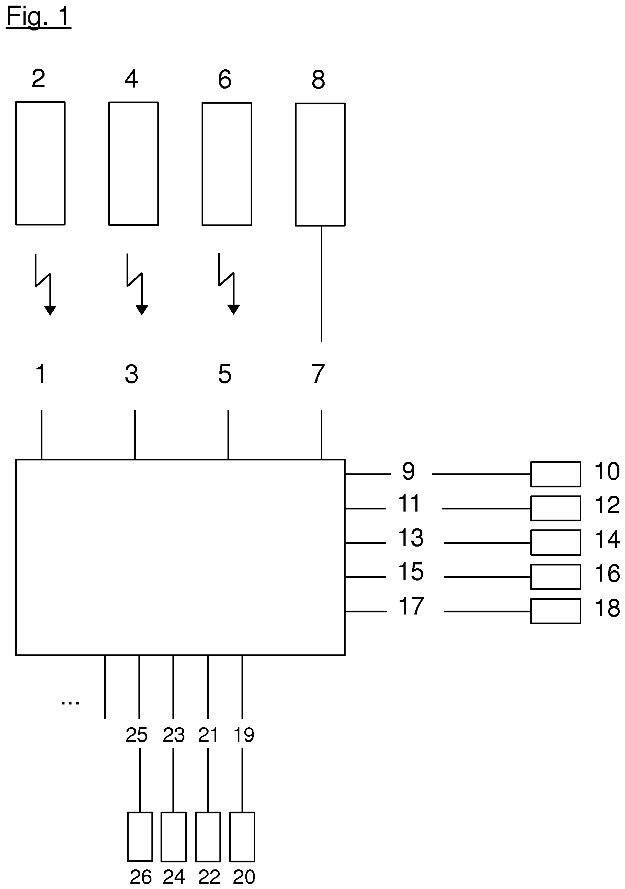

- Fig. 1: eine schematische Darstellung des Steuergerätes und seine Verbindung zu Sensoren und Aktoren.

Das in der Fig. 1 dargestellte Steuergerät S umfasst im Wesentlichen einen Mikrokontroller µC, der verschiedene Eingänge, Ausgänge, Funk-Schnittstellen und Bus-Schnittstellen hat.

So weist der Mikrocontroller µC eine EnOcean-Schnittstelle 1 auf, über die eine bidirektionale Kommunikation mit einem EnOcean-Sensor 2 möglich ist. Über Bedienelemente des Sensors könnten zum Beispiel Motoren zur Einstellung der Liegefläche oder von Teilen der Liegefläche und Leuchten des Bettes angesteuert werden.

Ferner weist der Mikrocontroller µC eine Bluetooth-Schnittstelle 3 auf, mit der eine Kommunikation mit einem dafür eingerichteten Smartphone 4 möglich ist, um zum Beispiel Motoren zur Einstellung der Liegefläche oder von Teilen der Liegefläche und Leuchten des Bettes, zum Beispiel einer Unterbettbeleuchtung ein- und auszuschalten.

Über eine WLAN-Schnittstelle 5 könnte der Mikrocontroller µC ebenfalls mit dem Smartphone 4 aber auch mit einem Tablet-Computer 6 oder dergleichen verbunden sein.

Sowohl das Smartphone 4 als auch der Tablet- Computer 6 könnten aber auch als Aktor genutzt werden, nämlich als Anzeige zum Darstellen von Informationen, zum Beispiel der Stellung der Liegefläche, dem Schaltzustand von Leuchten, eines Waageergebnisses, der Stellung von Seitensicherungen, der Raumtemperatur, der Raumhelligkeit, dem Öffnungszustand von Türen und Fenstern usw.

Über die WLAN- oder die Bluetooth-Schnittstellen 3, 5 könnten auch Alarmierungen oder Rufe nach dem Pflegepersonal von einem Bettinsassen an ein Smartphone 4 einer Pflegekraft, einen Tablet-Computer 6 einer Pflegekraft oder eine Rufanalage übertragen werden.

Neben diesen Funk-Schnittstellen weist der Mikrocontroller µC Bus-Schnittstellen auf, an die Busleitungen, insbesondere KNX-Leitungen oder Ethernet-Leitungen angeschlossen werden können, um zum Beispiel von Sensoren Informationen zu erhalten oder Aktoren zu steuern. So kann die Rufanlage 8 zum Beispiel auch über eine Ethernet-Schnittstelle 7 mit dem Bett verbunden sein.

Neben den Schnittstellen 1, 3, 5, 7 weist der Mikrocontroller µC auch einige Eingängen und Ausgänge auf.

Über Eingänge ist der Mikrocontroller µC mit weiteren Sensoren und Aktoren verbunden.

Ein Eingang 9 ist beispielsweise mit einer Waage 10 verbunden, die integraler Teil des Bettes ist, das mit dem erfindungsgemäßen Steuergerät ausgestatte ist.

Ein Eingang 11 ist mit einer Sensormatte 12 verbunden, mit der die Belastung einer Liegefläche erfasst werden kann, um festzustellen, ob sich eine Person im Bett befindet oder nicht.

Für den gleichen Zweck könnte ein Eingang 13 mit Drucksensoren 14 des Bettes verbunden sein.

Über einen Eingang 15 kann das Steuergerät mit einem Alarmknopf 16 verbunden werden. Über einen Ausgang 17 kann die Alarmierung einer Alarmierungseinrichtung 18 erfolgen.

Ein Notschalter 20 kann über einen Eingang 19 mit dem Mikrocontroller µC verbunden sein. Durch eine Betätigung des Notschalters 20 kann erreicht werden, dass die Motoren zum Einstellen der Liegefläche oder von Teilen der Liegefläche stillgesetzt werden. Das entsprechende Signal des Notschalters wird von dem Mikrocontroller µC verarbeitet und ein Signal zur Ansteuerung der Motoren erzeugt, das von dem Steuergerät S über Ausgänge 21 an die Motoren 22 gegeben wird.

Über weitere Eingänge 23 ist ein Handschalter 24 mit dem Mikrocontroller µC verbunden. Der Handschalter kann insbesondere zur Einstellung der Liegefläche oder Teilen der Liegefläche benutzt werden. Der Mikrocontroller µC nimmt dazu die Anweisung von dem Handschalter 24 auf und erzeugt die erforderlichen Signale zur Ansteuerung der Motoren 22, die über die Ausgänge 21 an die Motoren gegeben werden.

Über einen Ausgang 25 ist die Leuchte 26 einer Unterbettbeleuchtung an die Steuerung S angeschlossen.

## Patentansprüche

1. Steuergerät (S) für ein Bett, zum Steuern von Aktoren, insbesondere innerhalb und außerhalb des Bettes, zum Beispiel
- Motoren (22) zum Einstellen der Liegefläche oder von Teilen der Liegefläche, insbesondere zum Einstellen der Höhe oder der Neigung,
- ein Motor einer Jalousie, eines Rollos, eines Tür- oder Fensteröffners,
- eine Leuchte, insbesondere eine Leuchte (26) einer Unterbettbeleuchtung, eine Leseleuchte, eine Deckenleuchte oder eine Wandleuchte
- eine optische oder akustische Rufeinrichtung (8),
- ein Anzeigemittel, insbesondere ein Bildschirm,
- Radios oder Fernsehgeräte,
- Telefone (4) oder anderer Telekommunikationseinrichtungen,
- Computer (6) und Computerperipheriegeräten,
wobei das Steuergerät einen Mikrocontroller (µC) aufweist, der wenigstens eine Schnittstelle (1, 3, 5, 7) zur Kommunikation mit Sensoren und Aktoren (2, 4, 6, 8) und/oder wenigstens einen Eingang (9, 11, 13, 15, 19, 23) zur Kommunikation mit Sensoren (10, 12, 14, 16, 20, 24) und/oder wenigstens einen Ausgang (17, 21, 25) zur Kommunikation mit Aktoren (18, 22, 26) aufweist.

2. Steuergerät nach Anspruch 1, **dadurch gekennzeichnet, dass** wenigstens eine Schnittstelle (1, 3, 5) eine Funk-Schnittstelle, insbesondere eine EnOcean-Schnittstelle (1), eine Bluetooth-Schnittstelle (3) oder eine WLAN-Schnittstelle (5) ist.

3. Steuergerät nach Anspruch 1 oder 2, **dadurch gekennzeichnet, dass** der Mikrocontroller wenigstens eine Bus-Schnittstelle, insbesondere eine CAN-Schnittstelle, eine Ethernet-Schnittstelle (7) oder eine LIN-Schnittstelle aufweist.

4. Steuergerät nach Anspruch 3, **dadurch gekennzeichnet, dass** an die wenigstens eine Busschnittstelle ein Funkkommunikationsmodul, zum Beispiel ein EnOcean, Bluetooth oder WLAN-Modul des Steuergerätes angeschlossen ist, an den ein Sensor oder ein Aktor anschließbar ist.

5. Steuergerät nach einem der Ansprüche 1 bis 4, **dadurch gekennzeichnet, dass** an wenigstens eine der Schnittstellen ein Eingangsmodul mit einem Eingang und/oder Ausgangsmodul mit einem Ausgang anschließbar ist.

6. Steuergerät nach einem der Ansprüche 1 bis 4, **dadurch gekennzeichnet, dass** der an die wenigstens eine Schnittstelle oder den Eingang (3, 15, 19, 23) anschließbare Sensor ein Bedienelement (4, 16, 20, 24), eine Sensoreinrichtung, eine Bedieneinrichtung, insbesondere ein Handschalter (24) oder ein Smartphone (4), oder ähnliches ist.

7. Steuergerät nach Anspruch 6, **dadurch gekennzeichnet, dass** die Sensoreinrichtung eine Waage (10), eine Sensormatte (12), ein Positionssensor, ein Kraftsensor oder ein Druckschalter (14) ist.

8. Steuergerät nach einem der Ansprüche 1 bis 7, **dadurch gekennzeichnet, dass** der Sensor oder einer der Sensoren ein Helligkeitssensor ist.

9. Steuergerät (S) nach einem der Ansprüche 1 bis 8, **dadurch gekennzeichnet, dass** das Bedienelement ein Notschalter (20) ist, der über einen Eingang (19) oder eine Busschnittstelle (7) mit dem Steuergerät (S) verbindbar ist und mit dem ein aufgrund eines über die Funk-Schnittstelle oder eine der Funk-Schnittstellen empfangenes Sensor- oder Bediensignal vom Mikrocontroller erzeugten Signals für einen Aktor, insbesondere für einen Motor zum Einstellen der Liegefläche oder von Teilen der Liegefläche deaktivierbar ist.

10. Steuergerät (S) nach einem der Ansprüche 1 bis 9, **dadurch gekennzeichnet,**
- **dass** mit dem Steuergerät (S), insbesondere mit dem Mikrocontroller (µC), ein Aktorsignal erzeugbar ist, um eine an einen Ausgang oder eine Bus-Schnittstelle (7) angeschlossene Unterbettbeleuchtung (26) einzuschalten, sobald eine Sensormatte (12), eine Waage (10), ein Kraftsensor oder ein Druckschalter (14), die bzw. der an einen Eingang, eine Funk-Schnittstelle oder eine Bus-Schnittstelle angeschlossen ist, eine Entlastung detektiert und
- **dass** mit dem Steuergerät (S), insbesondere mit dem Mikrocontroller (µC), ein Aktorsignal erzeugbar ist, um eine an einen Ausgang oder eine Bus-Schnittstelle angeschlossenen Unterbettbeleuchtung (26) auszuschalten, sobald die Sensormatte (12), die Waage (10), der Kraftsensor oder der Druckschalter (14) eine Belastung detektiert.

11. Steuergerät (S) nach einem der Ansprüche 1 bis 10, **dadurch gekennzeichnet,**
- **dass** mit dem Steuergerät (S), insbesondere mit dem Mikrocontroller (µC), ein Aktorsignal erzeugbar ist, um eine an einen Ausgang oder eine Bus-Schnittstelle angeschlossene Unterbettbeleuchtung (26) einzuschalten, sobald ein Sensor, der an einen Eingang, eine Funk-Schnittstelle oder eine Bus-Schnittstelle angeschlossen ist, ein Absenken einer Seitensicherung des Bettes detektiert und
- **dass** mit dem Steuergerät (S), insbesondere mit dem Mikrocontroller (µC), ein Aktorsignal erzeugbar ist, um eine an einen Ausgang oder eine Bus-Schnittstelle angeschlossene Unterbettbeleuchtung (26) auszuschalten, sobald der Sensor ein Hochziehen der Seitensicherung des Bettes detektiert.

12. Steuergerät (S) nach Anspruch 10 oder 11, **dadurch gekennzeichnet, dass** bei entlasteter Sensormatte (12), entlasteter Waage (10), entlastetem Kraftsensor, entlastetem Druckschalter (14) oder abgesenkter Seitensicherung ein Einschalten der Unterbettbeleuchtung (26) durch das Steuergerät (S), insbesondere mit dem Mikrocontroller (µC), unterbleibt, wenn ein Helligkeitssensor eine Helligkeit oberhalb eines festgelegten Schwellwerts meldet oder signalisiert.

13. Steuergerät nach einem der Ansprüche 1 bis 12, **dadurch gekennzeichnet, dass** der wenigstens eine Eingang und/oder der wenigstens eine Ausgang des Steuergerätes (S) konfigurierbar ist.

14. Steuergerät (S) nach einem der Ansprüche 1 bis 13, **dadurch gekennzeichnet, dass** konfigurierbar ist, welche Aktoren und Sensoren über Schnittstellen mit dem Steuergerät (S) verbindbar oder verbunden sind.

15. Bett mit einer Liegefläche, deren Höhe und/oder Neigung einstellbar ist und/oder mit Teilen der Liegefläche deren Neigung einstellbar ist, wobei das Bett zum Einstellen der Liegefläche und/oder der Teile der Liegefläche Motoren (22) als Aktoren aufweist und das Bett wenigstens einen weiteren Aktor aufweist, und mit Sensoren, **dadurch gekennzeichnet, dass** das Bett ein Steuergerät (S) aufweist, mit dem die Aktoren des Bettes und die Sensoren des Bett verbunden sind, wobei das Steuergerät (S) nach einem der Ansprüche 1 bis 14 gestaltet ist.
